# EUROPEAN PATENT APPLICATION

(11) **EP 4 371 591 A2**
(43) Date of publication of application: **22.05.2024**
(21) Application number: 24155532.5
(22) Date of filing: 26.08.2020
(51) Int. Cl.: A61M 1/36

(54) **DRUG PERFUSION INTO AN UNARRESTED BEATING HEART**

(30) Priority: 27.08.2019 US 201962892164 P
(62) Divisional of application: 20775396.3
(71) Applicant: Dinaqor AG, 8952 Schlieren (CH)
(72) Inventor: HOLZMEISTER, Johannes, London, SW3 5HJ (GB); RICOTTI, Valeria, London, E14 9RU (GB)
(74) Representative: Maiwald GmbH

(57) **Abstract**

Disclosed is a method for treating a heart condition by perfusing a drug to an unarrested beating heart of a patient. A closed circuit may be formed with a first drug delivery catheter positioned in the patient's right coronary artery, a second drug delivery catheter positioned in a patient's left main coronary artery, a drug collection catheter positioned in a coronary sinus, the coronary artery, the coronary venous system, and an external membrane oxygenator interspersed between the venous and arterial branches. A drug for treating a heart condition may be perfused through the closed circuit.

## Description

### CROSS-REFERENCE TO RELATED APPLICATION(S)

This application claims the benefit of priority of U.S. Provisional Patent Application Serial No. 62/892,164, filed on August 27, 2019, the disclosure of which is hereby incorporated by reference herein in its entirety.

### FIELD OF THE INVENTION

The present invention relates to treatment of cardiac diseases, and, in particular, to localized delivery of therapeutic agents to a patient's heart.

### BACKGROUND OF THE INVENTION

Despite pharmacologic advances in the treatment of various heart conditions, such as heart failure, mortality, and morbidity remain unacceptably high. Furthermore, certain therapeutic approaches are not suitable for many patients (e.g., ones who have an advanced heart failure condition associated with other co-morbid diseases). Alternative approaches, such as gene therapy and cell therapy, have attracted increased attention due to their potential to be uniquely tailored and efficacious in addressing the root cause pathogenesis of many cardiac diseases.

Nevertheless, issues related to delivery, including vector efficiency, dose, specificity, and safety remain. As such, there is a need for further research directed to ways of achieving a more targeted, homogenous delivery of drugs suitable for treatment of various heart conditions that are also effective, well tolerated, and minimally invasive.

### OBJECTS AND SUMMARY OF THE INVENTION

It is an object of the present invention to provide methods for perfusing a drug in an unarrested beating heart of a patient in a minimally invasive manner.

It is an object of the present invention to provide methods for circulating a perfusate (which may contain one or more of blood or a drug) through an unarrested beating heart of a patient such that the perfusate is isolated from the patient's systemic circulation.

It is an object of the present invention to provide loco-regional delivery of pharmacogene therapy.

It is an object of the present invention to reduce the overall dose of a drug delivered to a patient for treating a heart condition.

It is an object of the present invention to reduce risks and/or adverse immune response to the administration of a drug suitable for treatment of a heart condition.

It is an object of the present invention to allow for re-dosing and/or dosing a pharmacogene therapy drug to patients who possess neutralizing antibodies that would otherwise be unsuitable candidates for receiving such drugs.

The above objects and others are met by the present invention which in certain embodiments are directed to a method of perfusing a drug in an unarrested beating heart of a patient. In some embodiments, the method comprises positioning a first drug delivery catheter in the right coronary artery of the heart. The method further comprises positioning a second drug delivery catheter in the left main coronary artery of the heart. The method further comprises positioning a drug collection catheter in the coronary sinus of the heart. In some embodiments, the first drug delivery catheter, the second drug delivery catheter, and the drug collection catheter together with the coronary arteries of the heart, the coronary venous system of the heart, and a membrane oxygenation device form a closed circuit. The method further comprises perfusing the drug through the closed circuit, which isolates the coronary circulation of the patient from the systemic circulation of the patient.

In some embodiments, the method further comprises applying negative pressure at the drug collection catheter. In some embodiments, the negative pressure ranges from about -100 mmHg to 0 mmHg.

In some embodiments, the closed circuit may further include one or more suction mechanisms allowing to further apply negative suction pressure to the drug collection catheter to prevent and/or minimize leakage of blood and/or drug circulated through the closed circuit through the Thebesian veins.

In some embodiments, one or more of the first drug delivery catheter, the second drug delivery catheter, or the drug collection catheter are introduced percutaneously. In some embodiments, the first drug delivery catheter and/or the second drug delivery catheter are positioned via antegrade intubation. In some embodiments, first drug delivery catheter and/or the second drug delivery catheter are positioned via the aorta of the patient by accessing the aorta femoralis and/or the aorta radialis. In some embodiments, the drug collection catheter is positioned in the coronary sinus via the vena cava of the patient. In some embodiments, the drug collection catheter is positioned via the vena jugularis of the patient or the vena femoralis. In some embodiments, the membrane oxygenation device is positioned between the collection catheter and one or more of the first drug delivery catheter and the second drug delivery catheter. In some embodiments, one or more of the first drug delivery catheter, the second drug delivery catheter, or the drug collection catheter are sealed by a balloon to reduce or prevent leakage.

In some embodiments, the method further comprises circulating blood through the closed circuit. In some embodiments, the blood comprises autologous blood, matched blood from donors, or a combination thereof. In some embodiments, blood components such as serum or plasma are chosen according to one or more parameters. In some embodiments, the one or more parameters comprise presence or absence of selected antibodies. In some embodiments, about 1000 mL, about 800 mL, about 600 mL, about 400 mL, about 200 mL, about 100 mL, or about 50 mL of blood is circulated through the closed circuit.

In some embodiments, the perfusing occurs over a duration of about 5 minutes to about 5 hours, about 15 minutes to about 4 hours, about 30 minutes to about 3 hours, or about 1 hour to about 2 hours. In some embodiments, the perfusing occurs for at least 60 minutes. In some embodiments, the perfusing occurs at a flow rate of about 75 mL/min to about 750 mL/min, about 150 mL/min to about 500 mL/min, or about 200 mL/min to about 300 mL/min.

In some embodiments, the drug is suitable for treatment of a heart condition. In some embodiments, the heart condition is heart failure. In some embodiments, the heart condition is a genetically determined heart disease. In some embodiments, the genetically determined heart disease is a genetically determined cardiomyopathy.

In some embodiments, the drug comprises a therapeutic polynucleotide sequence. In some embodiments, the therapeutic polynucleotide sequence is present in one or more viral vectors. In some embodiments, the one or more viral vectors is selected from the group consisting of an adeno-associated virus, an adenovirus, a retrovirus, a herpes simplex virus, a bovine papilloma virus, a lentiviral vector, a vaccinia virus, a polyoma virus, a sendai virus, orthomyxovirus, paramyxovirus, papovavirus, picornavirus, pox virus, alphavirus, variations thereof, and combinations thereof.

In some embodiments, the viral vector is an adeno-associated virus (AAV). In some embodiments, the AAV is one or more of AAV1, AAV2, AAV3, AAV4, AAV5, AAV6, AAV7, AAV8, AAV9, AAV10, AAV11, AAV12, variations thereof, and combinations thereof.

In some embodiments, the therapeutic polynucleotide sequence comprises a nucleic acid sequence encoding to a protein, antisense RNA, ncRNA, or miRNA for treatment of a heart condition. In some embodiments, the protein corresponds to a gene expressed in a human heart. In some embodiments, the protein is one or more of SERCA2, MyBPC3, MYH7, PKP2, dystrophin, FKRP, or a combination or variation thereof. In some embodiments, the therapeutic polynucleotide sequence comprises a promoter.

In some embodiments, less than about 20% v/v, less than about 15% v/v, less than about 10% v/v, less than about 5% v/v, less than about 4% v/v, less than about 3% v/v, less than about 2% v/v, less than about 1% v/v, less than about 0.5% v/v, or substantially no (0% v/v) blood circulated through the closed circuit leaks outside of the closed circuit. In some embodiments, less than about 20% v/v, less than about 15%v/v, less than about 10% v/v, less than about 5% v/v, less than about 4% v/v, less than about 3% v/v, less than about 2% v/v, less than about 1% v/v, less than about 0.5% v/v, or substantially no (0% v/v) drug perfused through the closed circuit leaks outside of the closed circuit.

In some embodiments, one or more of the first drug delivery catheter, the second drug delivery catheter, or the drug collection catheter is a balloon catheter.

The above objects and others are further met by the present invention which in certain embodiments are directed to a method of maintaining perfusion of a perfusate through a closed circuit in a heart of a patient that is unarrested and beating during the perfusion. In some embodiments, the method comprises positioning a first catheter in the right coronary artery of the heart. In some embodiments, the method further comprises positioning a second catheter in the left main coronary artery of the heart. In some embodiments, the method further comprises positioning a collection catheter in the coronary sinus of the heart. In some embodiments, the first catheter, the second catheter, and the collection catheter together with the coronary arteries, the coronary venous system, and a membrane oxygenation device form the closed circuit through the heart. In some embodiments, the method further comprises flowing the perfusate through the closed circuit by introducing the perfusate into the heart via the first catheter and the second catheter and collecting the perfusate via the collection catheter. In some embodiments, the closed circuit isolates the coronary circulation of the patient from the systemic circulation of the patient.

In some embodiments, the perfusion is maintained for at least 60 minutes. In some embodiments, the perfusion is maintained for at least 120 minutes.

In some embodiments, the method further comprises applying negative pressure at the collection catheter, wherein the negative pressure ranges from about -100 mmHg to 0 mmHg.

In some embodiments, one or more of the first catheter, the second catheter, or the collection catheter are introduced percutaneously.

In some embodiments, the membrane oxygenation device is positioned between the collection catheter and one or more of the first drug delivery catheter and the second drug delivery catheter.

In some embodiments, the method further comprises circulating blood through the closed circuit, wherein the blood comprises autologous blood, matched blood from donors, or a combination thereof. In some embodiments, about 1000 mL, about 800 mL, about 600 mL, about 400 mL, about 200 mL, about 100 mL, or about 50 mL of blood is circulated through the closed circuit.

In some embodiments, the perfusing occurs at a flow rate of about 75 mL/min to about 750 mL/min, about 150 mL/min to about 500 mL/min, or about 200 mL/min to about 300 mL/min. In some embodiments, less than about 20% v/v, less than about 15% v/v, less than about 10% v/v, less than about 5% v/v, less than about 4% v/v, less than about 3% v/v, less than about 2% v/v, less than about 1% v/v, less than about 0.5% v/v, or substantially no (0% v/v) blood circulated through the closed circuit leaks outside of the closed circuit.

In some embodiments, one or more of the first catheter, the second catheter, or the collection catheter is a balloon catheter.

The above objects and others are further met by the present invention which in certain embodiments are directed to a system for performing loco-regional perfusion within the heart of a patient when fluidly coupled thereto. In some embodiments, the system comprises: a first catheter adapted for insertion into the right coronary artery of the heart; a second catheter adapted for insertion into the left main coronary artery of the heart; a collection catheter adapted for insertion into the coronary sinus of the heart; a membrane oxygenation device fluidly coupled to the first catheter, the second catheter, the collection catheter, and an oxygen source; and a pump configured to drive fluid flow through the first catheter and the second catheter. In some embodiments, the first catheter, the second catheter, the collection catheter, and the membrane oxygenation device together form a closed circuit through the heart that is isolated from the patient's systemic circulation when the first catheter is inserted into the right coronary artery, the second catheter is inserted into the left main coronary artery, and the collection catheter is inserted into the coronary sinus.

The above objects and others are further met by the present invention which in certain embodiments are directed to a loco-regional perfusion system comprising: a first catheter inserted into the right coronary artery of a heart of a patient; a second catheter inserted into the left main coronary artery of the heart; a collection catheter inserted into the coronary sinus of the heart; a membrane oxygenation device fluidly coupled to the first catheter, the second catheter, the collection catheter, and an oxygen source; and a pump configured to drive fluid flow into the heart via the first catheter and the second catheter and out of the heart via the collection catheter. In some embodiments, the first catheter, the second catheter, the collection catheter, and the membrane oxygenation device together with the coronary arteries and the coronary venous system of the heart form a closed circuit through the heart that is isolated from the patient's systemic circulation.

In some embodiments, the membrane oxygenation device comprises a reservoir configured for injecting a drug into the closed circuit during perfusion.

In some embodiments, the pump is configured to generate negative pressure ranges from about -100 mmHg to 0 mmHg.

In some embodiments, one or more of the first catheter, the second catheter, or the collection catheter are introduced percutaneously. In some embodiments, the first catheter and/or the second catheter are positioned via antegrade intubation. In some embodiments, the collection catheter is positioned in the coronary sinus via the vena cava of the patient.

The above objects and others are further met by the present invention which in certain embodiments are directed to a loco-regional perfusion system configured to perform any of the aforementioned methods.

### BRIEF DESCRIPTION OF THE DRAWINGS

The above and other features of the present disclosure, their nature, and various advantages will become more apparent upon consideration of the following detailed description, taken in conjunction with the accompanying drawings, in which:
FIG. 1A depicts an exemplary loco-regional perfusion system in accordance with embodiments of the present disclosure;
FIG. 1B is a schematic of an exemplary loco-regional perfusion device in accordance with embodiments of the present disclosure;
FIG. 2 is a radiograph captured during loco-regional perfusion of an unarrested pig heart showing the locations of a left main coronary artery catheter, a right coronary artery catheter, and a coronary sinus balloon; and
FIG. 3 is a plot of pump speed, flow rate, and pressure measured during loco-regional perfusion.

### DEFINITIONS

As used herein, the singular forms "a," "an," and "the" include plural references unless the context clearly indicates otherwise. Thus, for example, reference to "a drug" includes a single drug as well as a mixture of two or more different drugs; and reference to a "viral vector" includes a single viral vector as well as a mixture of two or more different viral vectors, and the like.

Also as used herein, "about," when used in connection with a measured quantity, refers to the normal variations in that measured quantity, as expected by one of ordinary skill in the art in making the measurement and exercising a level of care commensurate with the objective of measurement and the precision of the measuring equipment. In certain embodiments, the term "about" includes the recited number ± 10%, such that "about 10" would include from 9 to 11.

Also as used herein, "polynucleotide" has its ordinary and customary meaning in the art and includes any polymeric nucleic acid such as DNA or RNA molecules, as well as chemical derivatives known to those skilled in the art. Polynucleotides include not only those encoding a therapeutic protein, but also include sequences that can be used to decrease the expression of a targeted nucleic acid sequence using techniques known in the art (e.g., antisense, interfering, or small interfering nucleic acids). Polynucleotides can also be used to initiate or increase the expression of a targeted nucleic acid sequence or the production of a targeted protein within cells of the cardiovascular system. Targeted nucleic acids and proteins include, but are not limited to, nucleic acids and proteins normally found in the targeted tissue, derivatives of such naturally occurring nucleic acids or proteins, naturally occurring nucleic acids or proteins not normally found in the targeted tissue, or synthetic nucleic acids or proteins. One or more polynucleotides can be used in combination, administered simultaneously and/or sequentially, to increase and/or decrease one or more targeted nucleic acid sequences or proteins.

Also as used herein, "perfusion," "perfused," and "perfusing" have their ordinary and customary meaning in the art and refer to administration for a time period (typically a minute or more) that is substantially longer than the art recognized term of "injection" or "bolus injection" (typically less than a minute). The flow rate of the perfusion will depend at least in part on the volume administered.

Also as used herein, "exogenous" nucleic acids or genes are those that do not occur in nature in the vector utilized for nucleic acid transfer; e.g., not naturally found in the viral vector, but the term is not intended to exclude nucleic acids encoding a protein or polypeptide that occurs naturally in the patient or host.

Also as used herein, "cardiac cell" includes any cell of the heart that is involved in maintaining a structure or providing a function of the heart such as a cardiac muscle cell, a cell of the cardiac vasculature, or a cell present in a cardiac valve. Cardiac cells include cardio myocytes (having both normal and abnormal electrical properties), epithelial cells, endothelial cells, fibroblasts, cells of the conducting tissue, cardiac pace making cells, and neurons.

Also as used herein, "isolated," "substantially isolated," "largely isolated," and their variants are terms that do not require complete or absolute isolation of the coronary venous, cardiac, systemic venous, or systemic circulation; rather, they are intended to mean that a majority, preferably the major part or even substantially all of the specified circulation is isolated. Also as used herein, "partially isolated" refers to any nontrivial portion of the specified circulation being isolated.

Also as used herein, "non-naturally restricted" includes any method of restricting the flow of fluid through a blood vessel, e.g., balloon catheter, sutures, etc., but does not include naturally occurring restriction, e.g., plaque build-up (stenosis). Non-natural restriction includes substantial or total isolation of, for example, the coronary circulation.

Also as used herein, "minimally invasive" is intended to include any procedure that does not require open surgical access to the heart or vessels closely associated with the heart. Such procedures include the use of endoscopic means to access the heart, and also catheter-based means relying on access via large arteries and veins.

Also as used herein, "adeno-associated virus" or "AAV" encompasses all subtypes, serotypes, and pseudotypes, as well as naturally occurring and recombinant forms. A variety of AAV serotypes and strains are known in the art and are publicly available from sources, such as the ATCC and academic or commercial sources. Alternatively, sequences from AAV serotypes and strains which are published and/or available from a variety of databases may be synthesized using known techniques.

Also as used herein, "serotype" refers to an AAV which is identified by and distinguished from other AAVs based on capsid protein reactivity with defined antisera. There are at least twelve known serotypes of human AAV, including AAV1 through AAV12, however additional serotypes continue to be discovered, and use of newly discovered serotypes are contemplated.

Also as used herein, "pseudotyped" AAV refers to an AAV that contains capsid proteins from one serotype and a viral genome including 5' and 3' inverted terminal repeats (ITRs) of a different or heterologous serotype. A pseudotyped recombinant AAV (rAAV) would be expected to have cell surface binding properties of the capsid serotype and genetic properties consistent with the ITR serotype. A pseudotyped rAAV may comprise AAV capsid proteins, including VP1, VP2, and VP3 capsid proteins, and ITRs from any serotype AAV, including any primate AAV serotype from AAV1 through AAV12, as long as the capsid protein is of a serotype heterologous to the serotype(s) of the ITRs. In a pseudotyped rAAV, the 5' and 3' ITRs may be identical or heterologous. Pseudotyped rAAV are produced using standard techniques described in the art.

Also as used herein, a "chimeric" rAAV vector encompasses an AAV vector comprising heterologous capsid proteins; that is, a rAAV vector may be chimeric with respect to its capsid proteins VP1, VP2, and VP3, such that VP1, VP2, and VP3 are not all of the same serotype AAV. A chimeric AAV as used herein encompasses AAV wherein the capsid proteins VP1, VP2, and VP3 differ in serotypes, including for example but not limited to capsid proteins from AAV1 and AAV2; are mixtures of other parvo virus capsid proteins or comprise other virus proteins or other proteins, such as for example, proteins that target delivery of the AAV to desired cells or tissues. A chimeric rAAV as used herein also encompasses an rAAV comprising chimeric 5' and 3'ITRs.

Also as used herein, a "pharmaceutically acceptable excipient or carrier" refers to any inert ingredient in a composition that is combined with an active agent in a formulation. A pharmaceutically acceptable excipient can include, but is not limited to, carbohydrates (such as glucose, sucrose, or dextrans), antioxidants (such as ascorbic acid or glutathione), chelating agents, low-molecular weight proteins, high-molecular weight polymers, gel-forming agents, or other stabilizers and additives. Other examples of a pharmaceutically acceptable carrier include wetting agents, emulsifying agents, dispersing agents, or preservatives, which are particularly useful for preventing the growth or action of microorganisms. Various preservatives are well known and include, for example, phenol and ascorbic acid. Examples of carriers, stabilizers or adjuvants can be found in Remington's Pharmaceutical Sciences, Mack Publishing Company, Philadelphia, Pa., 17th ed. (1985).

Also as used herein, a "patient" refers to a subject, particularly a human (but could also encompass a non-human), who has presented a clinical manifestation of a particular symptom or symptoms suggesting the need for treatment, who is treated prophylactically for a condition, or who has been diagnosed with a condition to be treated.

Also as used herein, a "subject" encompasses the definition of the term "patient" and does not exclude individuals who are otherwise healthy.

Also as used herein, "treatment of" and "treating" include the administration of a drug with the intent to lessen the severity of or prevent a condition, e.g., heart disease.

Also as used herein, "prevention of" and "preventing" include the avoidance of the onset of a condition, e.g., heart disease.

Also as used herein, a "condition" or "conditions" refers to those medical conditions, such as heart disease, that can be treated, mitigated, or prevented by administration to a subject of an effective amount of a drug.

Also as used herein, an "effective amount" refers to the amount of a drug that is sufficient to produce a beneficial or desired effect at a level that is readily detectable by a method commonly used for detection of such an effect. In some embodiments, such an effect results in a change of at least 10% from the value of a basal level where the drug is not administered. In other embodiments, the change is at least 20%, 50%, 80%, or an even higher percentage from the basal level. As will be described below, the effective amount of a drug may vary from subject to subject, depending on age, general condition of the subject, the severity of the condition being treated, the particular drug administered, and the like. An appropriate "effective" amount in any individual case may be determined by one of ordinary skill in the art by reference to the pertinent texts and literature and/or by using routine experimentation.

Also as used herein, an "active agent" refer to any material that is intended to produce a therapeutic, prophylactic, or other intended effect, whether or not approved by a government agency for that purpose.

Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to illuminate certain materials and methods and does not pose a limitation on scope. No language in the specification should be construed as indicating any non-claimed element as essential to the practice of the disclosed materials and methods.

### DETAILED DESCRIPTION

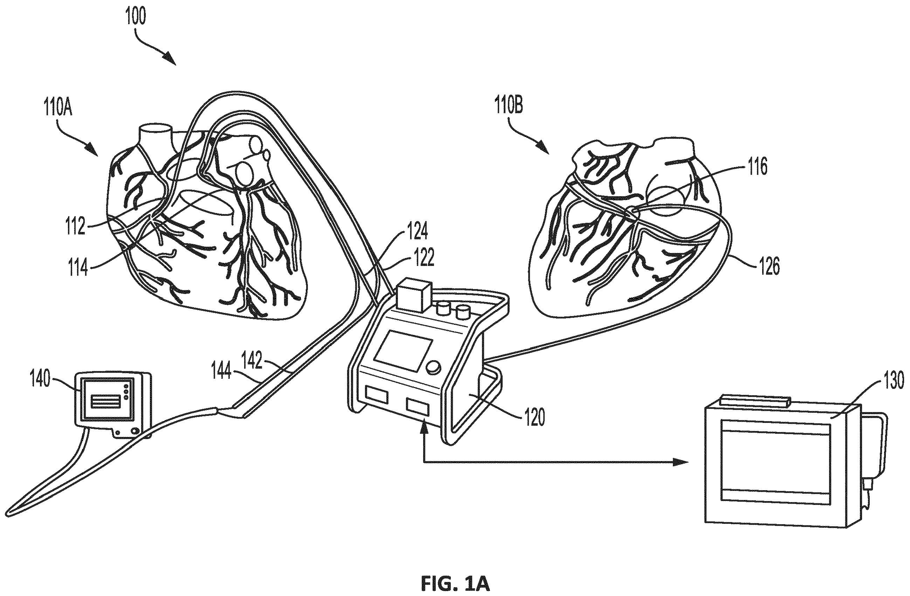

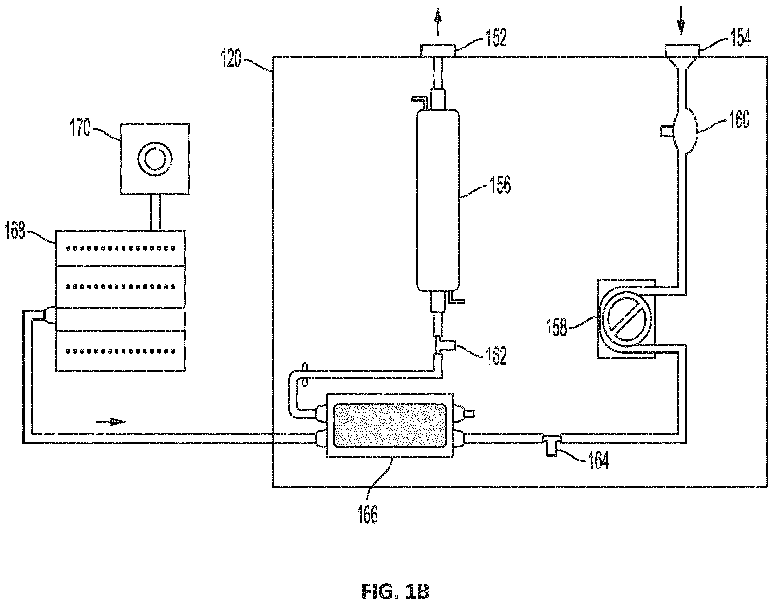

The present invention is directed to a method of treating a heart condition in a minimally invasive manner. The method may comprise, isolating a patient's coronary circulation from the patient's systemic circulation and perfusing a fluid, such as a drug-containing fluid, into the patient's isolated or substantially isolated coronary circulation. The perfusion may be performed into a patient's unarrested beating heart. Isolation of the patient's coronary circulation is described in more detail below with reference to FIGS. 1A and 1B.

The coronary circulation provides blood supply to the tissue of the heart. There are a number of coronary arteries. Normally, four main coronary arteries provide oxygenated blood to the heart for distribution throughout the heart tissue: the left main and right coronary arteries, the left anterior descending artery, and the left circumflex artery. Oxygen depleted blood flows through the coronary sinus.

Embodiments disclosed herein contemplate isolating or substantially isolating the coronary circulation of a patient from the systemic circulation of the patient by forming a closed circuit that comprises (consists of or consists essentially of) a first drug delivery catheter, a second drug delivery catheter, a drug collection catheter, a coronary artery, a coronary venous system, and an external membrane oxygenator. The instant disclosure further contemplates in certain embodiments perfusing a drug suitable for treatment of a heart condition to the heart muscle while substantially isolating the patient's coronary circulation from the patient's systemic circulation with the closed circuit described above. In some embodiments, the method disclosed herein delivers a drug to the heart muscle in its entirety as opposed to isolated regions within the heart. A drug delivered to the heart muscle with the methods disclosed herein may be distributed homogenously throughout the heart.

There are a number of advantages to isolating the coronary circulation of the patient from the systemic circulation of the patient when treating a heart condition. These advantages include, but are not limited to: (1) loco-regional delivery of the drug, minimal leakage of the drug to other organs, reduced overall drug dose; (2) increased targeted drug dose; (3) reduced risks and side-effects; and (4) possibility to re-dose select patients or to dose patient populations that were not suitable therapy candidates for certain therapies (such as gene therapy with viral vectors to patients who had antibodies to the viral vectors).

FIG. 1A depicts an exemplary loco-regional perfusion (LRP) system 100 in accordance with embodiments of the present disclosure. The LRP system 100 is shown in a closed circuit configuration with a heart 110 (with both an anterior view 110A and a posterior view 110B being shown for clarity). The LRP system 100 includes a membrane oxygenation device 120, a blood gas analysis (BGA) monitor 130, and a pressure monitor 140. The LRP system 100 may be assembled by positioning a first catheter 122 in the right coronary artery 112 of the heart 110, positioning a second catheter 114 in the left main coronary artery 114 of the heart 110, and positioning a collection catheter 126 in the coronary sinus 116 of the heart. The first catheter 122, the second catheter 124, and the collection catheter 126, together with the coronary arteries, the coronary venous system, the membrane oxygenation device 120, and one or more optional additional components form a closed circuit. This closed circuit may isolate or substantially isolate the coronary circulation of the patient from the systemic circulation of the patient.

The first catheter 122, the second catheter 124, and the collection catheter 126 may be introduced percutaneously and in a minimally invasive manner. In some embodiments, the first catheter 122 and/or the second catheter 124 may be introduced via antegrade intubation. In other embodiments, the first catheter 122 and/or the second catheter 124 may be introduced via retrograde intubation. The first catheter 122 and the second catheter 124 may be referred to herein as "drug delivery catheters" and the collection catheter 126 may be referred to herein as a "drug collection catheter" when the catheters are used for drug delivery to the heart.

The first catheter 122 and/or the second catheter 124 may be a standard infusion catheter that may optionally include a standard guidewire and infusion pump. Each catheter is capable of delivering a perfusate to the heart 110, which may contain, for example, a drug to be delivered to the heart 110 during loco-regional perfusion.

The first catheter 122 and/or the second catheter 124 may be positioned via the aorta of the patient, e.g., by accessing the aorta femoralis and/or the aorta radialis. In one embodiment, the first catheter 122 may be positioned via the aorta of the patient by accessing the aorta femoralis. In another embodiment, the first catheter 122 may be positioned via the aorta of the patient by accessing the aorta radialis. In one embodiment, the second catheter 124 may be positioned via the aorta of the patient by accessing the aorta femoralis. In another embodiment, the second catheter 122 may be positioned via the aorta of the patient by accessing the aorta radialis.

The collection catheter 126 may be a balloon catheter such that the balloon may be inflated within the coronary sinus 116 to ensure that all the blood circulated through the closed circuit flows through the collection catheter 126. The balloon catheter may be a Fogarthy^{®} catheter, or any other catheter suitable for the intended purpose discussed herein as will be appreciated by one of ordinary skill in the art. In certain embodiments, the collection catheter 126 may be positioned via the vena cava of the patient. In one embodiment, the collection catheter 126 may be positioned via the vena jugularis of the patient. In another embodiment, the collection catheter 126 may be positioned via the vena femoralis of the patient. In some embodiments, the first catheter 122, the second catheter 124, the collection catheter 126, or a combination thereof may each be a balloon catheter to help reduce leakage.

The LRP system 100 may further comprise one or more additional components, such as, without limitations, one or more pumps, one or more suction mechanisms, one or more perfusates, and combinations thereof. For example, the LRP system 100 is depicted as including a pressure monitor 140, which in some embodiments is operatively coupled to or part of the membrane oxygenation device 120. The pressure monitor 140 may be used to control the perfusion rate (i.e., flowrate) an ensure safety by continuously monitoring the coronary artery pressure. A first pressure sensor 142 and a second pressure sensor 144, for example, may be coinserted with the first catheter 112 and the second catheter 114, respectively, to measure the pressures within the right coronary artery and the left main coronary artery, respectively. The LRP system 100 is further depicted as including a BGA monitor that is operatively coupled to the membrane oxygenation device 120 to measure, for example, the gas concentrations in the perfusate (e.g., when the perfusate contains blood) prior to perfusion via the first catheter 122 and the second catheter 124 and/or after the perfusate is collected by the collection catheter 126. The membrane oxygenation device 120 and one or more additional components may be placed between the collection catheter 126 and one or more of the first catheter 122 or the second catheter 124.

FIG. 1B is a schematic of the membrane oxygenation device 120, which may be used to oxygenate the perfusate, mix the perfusate with other components (e.g., a drug), remove carbon dioxide from the perfusate, and/or push the perfusate into one or more of the first catheter 122 (in the right coronary artery 112) and/or the second catheter 124 (in the left main coronary artery 114). The membrane oxygenation device 120 may be any commercially available extracorporeal membrane oxygenation (ECMO) device for exchanging oxygen for carbon dioxide contained in the blood.

As illustrated in FIG. 1B, the membrane oxygenation device 120 includes various components including a heat exchanger 156 (through which the perfusate passes prior to leaving an outlet 152 and entering the first catheter 122 and the second catheter 124), a delivery pump 158, a reservoir 160 (for adding a component, such as blood and/or a drug, to the perfusate returning through the collection catheter 126 through an inlet 154), sensors 162 and 164 at various stages of the closed circuit (e.g., for measuring pressure and/or blood gas content), and a membrane oxygenator 166. In some embodiments, de-oxygenated blood enters the membrane oxygenator 166 and is mixed with an oxygen-rich gas. The oxygen-rich gas may be supplied from a gas blender 168 that may mix oxygen in various ratios with carbon dioxide and nitrogen gas, and is regulated by a gas regulator 170.

The perfusate may comprise one or more of blood (or its components such as plasma or serum) and/or drug suitable for treatment of the heart condition and/or a vehicle such as saline or dextrose solutions. The delivery pump 158 may deliver the perfusate into the first catheter 122 and/or the second catheter 124. In some embodiments, the perfusate may be contained in an IV bag or a syringe and may be administered directly to the first catheter 124 and/or the second catheter 124 with or without the delivery pump 158.

A suction mechanism may be used to apply negative suction pressure on the collection catheter 126 to minimize blood and/or drug leakage through the Thebesian venous system. The negative suction pressure may be about -150 mmHg, about -100 mmHg, about -50 mmHg, about -20 mmHg, about -15 mmHg, about -10 mmHg, about -5 mmHg, 0 mmHg, or within a subrange defined by any of these points.

Blood circulated through the closed circuit may be autologous blood, matched blood from donors, or a combination thereof. In some embodiments, blood components, such as serum or plasma, are chosen according to one or more parameters. One of the parameters may be the presence or absence of selected antibodies. For instance, when the drug is one or more viral vectors encompassing a therapeutic nucleic acid sequence, the patient's autologous blood may be screened to determine whether antibodies to the one or more viral vectors are present. Presence of antibodies in the patient's autologous blood may reduce and/or negate altogether the effectiveness of the treatment and/or may result in an undesirable immune response. As such, it may be possible to dilute or replace the patient's autologous blood with a seronegative matched blood from donors, thereby reducing a patient's immune response to the drug and enhancing the effectiveness of the drug.

While the various components illustrated in FIG. 1B show components that are part of or separate from the membrane oxygenation device 120, it is to be understood that this schematic is merely illustrative, as one or more of the components may be included in or separate (external) from the membrane oxygenation device 120.

The LRP system 100 may be set up and operated as follows: (1) the collection catheter 126 is carefully placed and tightly sealed in the coronary sinus 116 to enable the collection of the only cardiac venous (de-oxygenated) blood; (2) the first catheter 122 and the second catheter 124 are placed in the right coronary artery (RCA) and left main coronary artery (RCA) in a sealed fashion; (3) the catheters are then connected to arterial and venous lines of the membrane oxygenation device 120 using standard tubes; (4) operation of the LRP system 100 is started, and the coronary arteries are antegradely perfused with oxygenated blood, while the returning de-oxygenated blood is collected from the venous cardiac system via the collection catheter 126 using gentle negative pressure; (5) blood is then directed into the reservoir 160 and is subsequently oxygenated by the membrane oxygenator 166 and antegradely re-infused (driven by the delivery pump 160) into the heart via the first catheter 122 and the second catheter 124. If a drug (e.g., a vector) is administered, this can be added into the perfusate via the reservoir 160 after priming with blood or plasma, and blood samples can be taken, or drugs can be applied via the reservoir 160 during the entire perfusion process.

In some embodiments, diluting a patient's antibody-containing autologous blood with a seronegative matched blood from donors may result in a reduced adverse immune response and/or improved drug efficacy. For instance, the adversity of a patient's immune response may be reduced by about 10%, by about 20%, by about 30%, by about 40%, by about 50%, by about 60%, by about 70%, by about 80%, by about 90%, or alleviated altogether, upon dilution or replacement of autologous blood with seronegative matched blood from donors as compared to a patient's immune response without autologous blood dilution or replacement. The efficacy of a drug administered may be increased by about 10%, by about 20%, by about 30%, by about 40%, by about 50%, by about 60%, by about 70%, by about 80%, by about 90%, by about 100%, by about 150%, by about 200%, by about 300%, by about 400%, or by about 500%, upon dilution or replacement of autologous blood with seronegative matched blood from donors as compared to the drug's efficacy in a patient without autologous blood dilution or replacement.

In some embodiments, the blood portion of the perfusate may range from about 5 mL to about 5000 mL, from about 50 mL to about 2500 mL, from about 100 mL to about 1000 mL, from about 150 mL to about 500 mL, about 50 mL, about 75 mL, about 100 mL, about 125 mL, about 150 mL, about 175 mL, about 200 mL, about 225 mL, about 250 mL, about 275 mL, about 300 mL, about 325 mL, about 350 mL, about 375 mL, about 400 mL, about 425 mL, about 450 mL, about 475 mL, about 500 mL, about 550 mL, about 600 mL, about 650 mL, about 700 mL, about 750 mL, about 800 mL, about 850 mL, about 900 mL, about 950 mL, or about 1000 mL.

The ratio of autologous blood to blood matched from donors in the blood that is circulated through the closed circuit may be adjusted, as needed, to obtain a blood mixture that would be most receptive to the drug and would generate the least immune response upon introduction of the drug. In some embodiments the ratio may range from about 1:100 to about 100:1, from about 1:80 to about 80:1, from about 1:50 to about 50:1, from about 1:30 to about 30:1, from about 1:20 to about 20:1, from about 1:10 to about 10:1, from about 1:8 to about 8:1, from about 1:5 to about 5:1, from about 1:3 to about 3:1, or from about 1:2 to about 2:1 of (volume autologous blood) : (volume blood matched from donors).

The flow rate of the perfusate through the closed circuit may be adjusted to match the patient's blood flow rate. As appreciated by one of ordinary skill in the art, the blood flow rate varies from patient to patient, and for any given patient, varies throughout the day. Accordingly, the flow rate of the perfusate circulated through the closed circuit may be adjusted in situ. The flow rate may be measured over the closed circuit. In certain embodiments, the flow rate may be measured with a transonic probe (such as a clamp over tubing). In some embodiments, the flow rate of the perfusate, at any given time during the perfusion, may be within about 20%, within about 15%, within about 10%, within about 8%, within about 5%, within about 3%, within about 2%, within about 1%, or within about 0.5% of the patient's blood flow rate, based on mL/min units. It is important that the flow rate of the perfusate circulated through the closed circuit does not deviate significantly from the patient's own blood flow rate in order to avoid ischemia and/or under perfusion.

Exemplary flow rates for the perfusate circulated through the closed circuit may range, without limitations, from about 75 mL/min to about 750 mL/min, from about 100 mL/min to about 650 mL/min, from about 125 mL/min to about 600 mL/min, from about 150 mL/min to about 500 mL/min, from about 175 mL/min to about 400 mL/min, from about 200 mL/min to about 300 mL/min, about 150 mL/min, about 175 mL/min, about 200 mL/min, about 225 mL/min, about 250 mL/min, about 275 mL/min, about 300 mL/min, about 325 mL/min, or about 350 mL/min.

The perfusate may be circulated through the closed circuit for a duration ranging, without limitations, from about 5 minutes to about 5 hours, from about 15 minutes to about 4 hours, from about 30 minutes to about 3 hours, or from about 1 hour to about 2 hours. In some embodiments, the treatment duration may occur over the span of days, e.g., 1 day, 2 days, 3 days, 4 days, 5 days, 6 days, 7 days, and so on.

With the system disclosed herein, in some embodiments, a higher dose of drug than could otherwise be administered safely through systemic delivery may be administered directly and only to the heart. In some embodiments, a lower overall dose of drug may be required to attain the same therapeutic effect (as was attained with a larger dose that was subjected to systemic circulation or that was subjected to only partial isolation of the coronary circulation), since there may be substantially no leakage of the perfusate outside of the heart and/or to the Thebesian venous system.

In some embodiments, less than about 50% v/v, less than about 40% v/v, less than about 30% v/v, less than about 20% v/v, less than about 15% v/v, less than about 10% v/v, less than about 5% v/v, less than about 4% v/v, less than about 3% v/v, less than about 2% v/v, less than about 1% v/v, less than about 0.5% v/v, or substantially no (0% v/v) perfusate (e.g., blood and/or drug) circulated through the closed circuit leaks outside of the closed circuit during the perfusion process.

The reduced perfusate leakage outside of the closed circuit (as compared to other methods disclosed in the art) may be due to the tight seal formed within the closed circuit and each individual component utilized in the closed circuit.

In certain embodiments, some perfusate leakage from the closed circuit may remain. For instance, up to about 0.5% v/v, about 1% v/v, about 2% v/v, about 3% v/v, about 4% v/v, about 5% v/v, about 10% v/v, about 15% v/v, about 20% v/v, about 30% v/v, about 40% v/v, or about 50% v/v of the perfusate circulated through the closed circuit may leak outside of the closed circuit. Any drug amount lost through leakage of the perfusate may be replaced in the perfusate in order to keep the drug exposure to the heart constant over the calculated exposure time. The calculated exposure time may, in certain embodiments, range from about 5 minutes to about 5 hours, from about 15 minutes to about 4 hours, from about 30 minutes to about 3 hours, from about 1 hour to about 2 hours, or any sub-range in between.

### THERAPEUTIC COMPOSITIONS

Drugs suitable for treatment of the heart condition (i.e., drugs included in the perfusate) may include therapeutic polynucleotide sequences. In some embodiments, the therapeutic polynucleotide sequences may encode to a protein for the treatment of a heart condition. The protein for treatment of the heart condition may be of human origin or may be derived from different species (e.g., without limitations, mouse, cat, pig or monkey). In some embodiments, the protein encoded by the therapeutic polynucleotide sequence may correspond to a gene expressed in a human heart.

Exemplary proteins may include, without limitations, one or more of SERCA2, MYBPC3, MYH7, PKP2, MYL3, MYL2, ACTC1, TPM1, TNNT2, TNNI3, TTN, FHL1, ALPK3, dystrophin, FKRP, variants thereof, or combinations thereof. The protein or proteins used may also be functional variants of the proteins mentioned herein and may exhibit a significant amino acid sequence identity compared to the original protein. For instance, the amino acid identity may amount to at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%. In this context, the term "functional variant" means that the variant of the protein is capable of, partially or completely, fulfilling the function of the naturally occurring corresponding protein. Functional variants of a protein may include, for example, proteins that differ from their naturally occurring counterparts by one or more amino acid substitutions, deletions, or additions.

The amino acid substitutions can be conservative or non-conservative. It is preferred that the substitutions are conservative substitutions, i.e., a substitution of an amino acid residue by an amino acid of similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids, which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine, and lysine. Examples of charged polar (acidic) amino acids comprise aspartic acid and glutamic acid.

Also considered as variants are proteins that differ from their naturally occurring counterparts by one or more (e.g., 2, 3, 4, 5, 10, or 15) additional amino acids. These additional amino acids may be present within the amino acid sequence of the original protein (i.e., as an insertion), or they may be added to one or both termini of the protein. Basically, insertions can take place at any position if the addition of amino acids does not impair the capability of the polypeptide to fulfill the function of the naturally occurring protein in the treated subject. Moreover, variants of proteins also comprise proteins in which, compared to the original polypeptide, one or more amino acids are lacking. Such deletions may affect any amino acid position provided that it does not impair the ability to fulfill the normal function of the protein.

Finally, variants of the cardiac sarcomeric proteins also refer to proteins that differ from the naturally occurring protein by structural modifications, such as modified amino acids. Modified amino acids are amino acids which have been modified either by natural processes, such as processing or post-translational modifications, or by chemical modification processes known in the art. Typical amino acid modifications comprise phosphorylation, glycosylation, acetylation, O-linked N-acetylglucosamination, glutathionylation, acylation, branching, ADP ribosylation, crosslinking, disulfide bridge formation, formylation, hydroxylation, carboxylation, methylation, demethylation, amidation, cyclization, and/or covalent or non-covalent bonding to phosphotidylinositol, flavine derivatives, lipoteichonic acids, fatty acids, or lipids.

The therapeutic polynucleotide sequence encoding the target protein may be administered to the subject to be treated in the form of a gene therapy vector, i.e., a nucleic acid construct which comprises the coding sequence, including the translation and termination codons, next to other sequences required for providing expression of the exogenous nucleic acid such as promoters, kozak sequences, polyA signals, and the like.

For example, the gene therapy vector may be part of a mammalian expression system. Useful mammalian expression systems and expression constructs are commercially available. Also, several mammalian expression systems are distributed by different manufacturers and can be employed in the present invention, such as plasmid- or viral vector based systems, e.g., LENTI-Smart^{™} (InvivoGen), GenScript^{™} Expression vectors, pAdVAntage^{™} (Promega), ViraPower^{™} Lentiviral, Adenoviral Expression Systems (Invitrogen), and adeno-associated viral expression systems (Cell Biolabs).

Gene therapy vectors for expressing an exogenous therapeutic polynucleotide sequence of the invention can be, for example, a viral or non-viral expression vector, which is suitable for introducing the exogenous therapeutic polynucleotide sequence into a cell for subsequent expression of the protein encoded by said nucleic acid. The expression vector can be an episomal vector, i.e., one that is capable of self-replicating autonomously within the host cell, or an integrating vector, i.e., one which stably incorporates into the genome of the cell. The expression in the host cell can be constitutive or regulated (e.g., inducible).

In a certain embodiment, the gene therapy vector is a viral expression vector. Viral vectors for use in the present invention may comprise a viral genome in which a portion of the native sequence has been deleted in order to introduce a heterogeneous polynucleotide without destroying the infectivity of the virus. Due to the specific interaction between virus components and host cell receptors, viral vectors are highly suitable for efficient transfer of genes into target cells. Suitable viral vectors for facilitating gene transfer into a mammalian cell can be derived from different types of viruses, for example, from an AAV, an adenovirus, a retrovirus, a herpes simplex virus, a bovine papilloma virus, a lentivirus, a vaccinia virus, a polyoma virus, a sendai virus, orthomyxovirus, paramyxovirus, papovavirus, picornavirus, pox virus, alphavirus, or any other viral shuttle suitable for gene therapy, variations thereof, and combinations thereof.

"Adenovirus expression vector" or "adenovirus" is meant to include those constructs containing adenovirus sequences sufficient (a) to support packaging of the therapeutic polynucleotide sequence construct, and/or (b) to ultimately express a tissue and/or cell-specific construct that has been cloned therein. In one embodiment of the invention, the expression vector comprises a genetically engineered form of adenovirus. Knowledge of the genetic organization of adenovirus, a 36 kilobase (kb), linear, double-stranded DNA virus, allows substitution of large pieces of adenoviral DNA with foreign sequences up to 7 kb.

Adenovirus growth and manipulation is known to those of skill in the art, and exhibits broad host range in vitro and in vivo. This group of viruses can be obtained in high titers, e.g., 10⁹ to 10¹¹ plaque-forming units per mL, and they are highly infective. The life cycle of adenovirus does not require integration into the host cell genome. The foreign genes delivered by adenovirus vectors are episomal and, therefore, have low genotoxicity to host cells. No side effects have been reported in studies of vaccination with wild-type adenovirus, demonstrating their safety and/or therapeutic potential as in vivo gene transfer vectors.

Retroviruses (also referred to as "retroviral vector") may be chosen as gene delivery vectors due to their ability to integrate their genes into the host genome, transferring a large amount of foreign genetic material, infecting a broad spectrum of species and cell types and for being packaged in special cell-lines.

The retroviral genome contains three genes, gag, pol, and env that code for capsid proteins, polymerase enzyme, and envelope components, respectively. A sequence found upstream from the gag gene contains a signal for packaging of the genome into virions. Two long terminal repeat (LTR) sequences are present at the 5' and 3' ends of the viral genome. These contain strong promoter and enhancer sequences and are also required for integration in the host cell genome.

In order to construct a retroviral vector, a nucleic acid encoding a gene of interest is inserted into the viral genome in the place of certain viral sequences to produce a virus that is replication-defective. In order to produce virions, a packaging cell line is constructed containing the gag, pol, and/or env genes but without the LTR and/or packaging components. When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media. The media containing the recombinant retroviruses is then collected, optionally concentrated, and used for gene transfer. Retroviral vectors are able to infect a broad variety of cell types. However, integration and stable expression require the division of host cells.

The retrovirus can be derived from any of the subfamilies. For example, vectors from Murine Sarcoma Virus, Bovine Leukemia, Virus Rous Sarcoma Virus, Murine Leukemia Virus, Mink-Cell Focus-Inducing Virus, Reticuloendotheliosis Virus, or Avian Leukosis Virus can be used. The skilled person will be able to combine portions derived from different retroviruses, such as LTRs, tRNA binding sites, and packaging signals to provide a recombinant retrovirus. These retroviruses are then normally used for producing transduction competent retroviral vector particles. For this purpose, the vectors are introduced into suitable packaging cell lines. Retroviruses can also be constructed for site-specific integration into the DNA of the host cell by incorporating a chimeric integrase enzyme into the retroviral particle.

Because herpes simplex virus (HSV) is neurotropic, it has generated considerable interest in treating nervous system disorders. Moreover, the ability of HSV to establish latent infections in non-dividing neuronal cells without integrating into the host cell chromosome or otherwise altering the host cell's metabolism, along with the existence of a promoter that is active during latency makes HSV an attractive vector. And though much attention has focused on the neurotropic applications of HSV, this vector also can be exploited for other tissues given its wide host range.

Another factor that makes HSV an attractive vector is the size and organization of the genome. Because HSV is large, incorporation of multiple genes or expression cassettes is less problematic than in other smaller viral systems. In addition, the availability of different viral control sequences with varying performance (temporal, strength, etc.) makes it possible to control expression to a greater extent than in other systems. It also is an advantage that the virus has relatively few spliced messages, further easing genetic manipulations.

HSV also is relatively easy to manipulate and can be grown to high titers. Thus, delivery is less of a problem, both in terms of volumes needed to attain sufficient multiplicity of infection (MOI) and in a lessened need for repeat dosing. Avirulent variants of HSV have been developed and are readily available for use in gene therapy contexts.

Lentiviruses are complex retroviruses, which, in addition to the common retroviral genes gag, pol, and env, contain other genes with regulatory or structural function. The higher complexity enables the virus to modulate its life cycle, as in the course of latent infection. Some examples of lentivirus include the Human Immunodeficiency Viruses (HIV-1, HIV-2) and the Simian Immunodeficiency Virus (SIV). Lentiviral vectors have been generated by multiply attenuating the HIV virulence genes, for example, the genes env, vif, vpr, vpu, and nef are deleted making the vector biologically safe.

Lentiviral vectors are plasmid-based or virus-based, and are configured to carry the essential sequences for incorporating foreign nucleic acid, for selection and for transfer of the nucleic acid into a host cell. The gag, pol and env genes of the vectors of interest also are known in the art. Thus, the relevant genes are cloned into the selected vector and then used to transform the target cell of interest.

Vaccinia virus vectors have been used extensively because of the ease of their construction, relatively high levels of expression obtained, wide host range and large capacity for carrying DNA. Vaccinia contains a linear, double-stranded DNA genome of about 186 kb that exhibits a marked "A-T" preference. Inverted terminal repeats of about 10.5 kb flank the genome. The majority of essential genes appear to map within the central region, which is most highly conserved among poxviruses. Estimated open reading frames in vaccinia virus number from 150 to 200. Although both strands are coding, extensive overlap of reading frames is not common.

At least 25 kb can be inserted into the vaccinia virus genome. Prototypical vaccinia vectors contain transgenes inserted into the viral thymidine kinase gene via homologous recombination. Vectors are selected on the basis of a tk-phenotype. Inclusion of the untranslated leader sequence of encephalomyocarditis virus results in a level of expression that is higher than that of conventional vectors, with the transgenes accumulating at 10% or more of the infected cell's protein in 24 hours.

The empty capsids of papovaviruses, such as the mouse polyoma virus, have received attention as possible vectors for gene transfer. The use of empty polyoma was first described when polyoma DNA and purified empty capsids were incubated in a cell-free system. The DNA of the new particle was protected from the action of pancreatic DNase. The reconstituted particles were used for transferring a transforming polyoma DNA fragment to rat Fill cells. The empty capsids and reconstituted particles consist of all three of the polyoma capsid antigens VP1, VP2, and VP3.

AAVs are parvoviruses belonging to the genus Dependovirus. They are small, nonenveloped, single-stranded DNA viruses which require a helper virus in order to replicate. Co-infection with a helper virus (e.g., adenovirus, herpes virus, or vaccinia virus) is necessary in order to form functionally complete AAV virions. In vitro, in the absence of co-infection with a helper virus, AAV establishes a latent state in which the viral genome exists in an episomal form, but infectious virions are not produced. Subsequent infection by a helper virus "rescues" the genome, allowing it to be replicated and packaged into viral capsids, thereby reconstituting the infectious virion. Recent data indicate that in vivo both wild type AAV and recombinant AAV predominantly exist as large episomal concatemers. In one embodiment, the gene therapy vector used herein is an AAV vector. The AAV vector may be purified, replication incompetent, pseudotyped rAAV particles.

AAV are not associated with any known human diseases, are generally not considered pathogenic, and do not appear to alter the physiological properties of the host cell upon integration. AAV can infect a wide range of host cells, including non-dividing cells, and can infect cells from different species. In contrast to some vectors, which are quickly cleared or inactivated by both cellular and humoral responses, AAV vectors have been shown to induce persistent transgene expression in various tissues in vivo. The persistence of recombinant AAV-mediated transgenes in non-diving cells in vivo may be attributed to the lack of native AAV viral genes and the vector's ITR-linked ability to form episomal concatemers.

AAV is an attractive vector system for use in the cell transduction of the present invention as it has a high frequency of persistence as an episomal concatemer and it can infect non-dividing cells, including cardiomyocytes, thus making it useful for delivery of genes into mammalian cells, for example, in tissue culture and in vivo.

Typically, rAAV is made by cotransfecting a plasmid containing the gene of interest flanked by the two AAV terminal repeats and/or an expression plasmid containing the wild-type AAV coding sequences without the terminal repeats, for example pIM45. The cells are also infected and/or transfected with adenovirus and/or plasmids carrying the adenovirus genes required for AAV helper function. Stocks of rAAV made in such fashion are contaminated with adenovirus, which must be physically separated from the rAAV particles (for example, by cesium chloride density centrifugation or column chromatography). Alternatively, adenovirus vectors containing the AAV coding regions and/or cell lines containing the AAV coding regions and/or some or all of the adenovirus helper genes could be used. Cell lines carrying the rAAV DNA as an integrated provirus can also be used.

Multiple serotypes of AAV exist in nature, with at least twelve serotypes (AAV1-AAV12). Despite the high degree of homology, the different serotypes have tropisms for different tissues. Upon transfection, AAV elicits only a minor immune reaction (if any) in the host. Therefore, AAV is highly suited for gene therapy approaches.

The present disclosure may be directed in some embodiments to a drug comprising an AAV vector that is one or more of AAV1, AAV2, AAV3, AAV4, AAV5, AAV6, AAV7, AAV8, AAV9, AAV10, AAV11, AAV12, ANC AAV, chimeric AAV derived thereof, variations thereof, and combinations thereof, which will be even better suitable for high efficiency transduction in the tissue of interest. In certain embodiments, the gene therapy vector is an AAV serotype 1 vector. In certain embodiments, the gene therapy vector is an AAV serotype 2 vector. In certain embodiments, the gene therapy vector is an AAV serotype 3 vector. In certain embodiments, the gene therapy vector is an AAV serotype 4 vector. In certain embodiments, the gene therapy vector is an AAV serotype 5 vector. In certain embodiments, the gene therapy vector is an AAV serotype 6 vector. In certain embodiments, the gene therapy vector is an AAV serotype 7 vector. In certain embodiments, the gene therapy vector is an AAV serotype 8 vector. In certain embodiments, the gene therapy vector is an AAV serotype 9 vector. In certain embodiments, the gene therapy vector is an AAV serotype 10 vector. In certain embodiments, the gene therapy vector is an AAV serotype 11 vector. In certain embodiments, the gene therapy vector is an AAV serotype 12 vector.

A suitable dose of AAV for humans may be in the range of about 1×10⁸ vg/kg to about 3×10¹⁴ vg/kg, about 1×10⁸ vg/kg, about 1×10⁹ vg/kg, about 1×10¹⁰ vg/kg, about 1×10¹¹ vg/kg, about 1×10¹² vg/kg, about 1×10¹³ vg/kg, or about 1×10¹⁴ vg/kg. The total amount of viral particles or DRP is, is about, is at least, is at least about, is not more than, or is not more than about, 5×10¹⁵ vg/kg, 4×10¹⁵ vg/kg, 3×10¹⁵ vg/kg, 2×10¹⁵ vg/kg, 1×10¹⁵ vg/kg, 9×10¹⁴ vg/kg, 8×10¹⁴ vg/kg, 7×10¹⁴ vg/kg, 6×10¹⁴ vg/kg, 5×10¹⁴ vg/kg, 4×10¹⁴ vg/kg, 3×10¹⁴ vg/kg, 2×10¹⁴ vg/kg, 1×10¹⁴ vg/kg, 9×10¹³ vg/kg, 8×10¹³ vg/kg, 7×10¹³ vg/kg, 6×10¹³ vg/kg, 5×10¹³ vg/kg, 4×10¹³ vg/kg, 3×10¹³ vg/kg, 2×10¹³ vg/kg, 1×10¹³ vg/kg, 9×10¹² vg/kg, 8×10¹² vg/kg, 7×10¹² vg/kg, 6×10¹² vg/kg, 5×10¹² vg/kg, 4×10¹² vg/kg, 3×10¹² vg/kg, 2×10¹² vg/kg, 1×10¹² vg/kg, 9×10¹¹ vg/kg, 8×10¹¹ vg/kg, 7×10¹¹ vg/kg, 6×10¹¹ vg/kg, 5×10¹¹ vg/kg, 4×10¹¹ vg/kg, 3×10¹¹ vg/kg, 2×10¹¹ vg/kg, 1×10¹¹ vg/kg, 9×10¹⁰ vg/kg, 8×10¹⁰ vg/kg, 7×10¹⁰ vg/kg, 6×10¹⁰ vg/kg, 5×10¹⁰ vg/kg, 4×10¹⁰ vg/kg, 3×10¹⁰ vg/kg, 2×10¹⁰ vg/kg, 1×10¹⁰ vg/kg, 9×10⁹ vg/kg, 8×10⁹ vg/kg, 7×10⁹ vg/kg, 6×10⁹ vg/kg, 5×10⁹ vg/kg, 4×10⁹ vg/kg, 3×10⁹ vg/kg, 2×10⁹ vg/kg, 1×10⁹ vg/kg, 9×10⁸ vg/kg, 8×10⁸ vg/kg, 7×10⁸ vg/kg, 6×10⁸ vg/kg, 5×10⁸ vg/kg, 4×10⁸ vg/kg, 3×10⁸ vg/kg, 2×10⁸ vg/kg, or 1×10⁸ vg/kg, or falls within a range defined by any two of these values. The above listed dosages being in vg/kg heart tissue units.

With the system and methods disclosed herein, in some embodiments, a higher dose of drug than could otherwise be administered safely through systemic delivery may be administered directly and only to the heart, since there is substantially no leakage of the perfusate outside of the heart and/or to the Thebesian venous system. Without being construed as limiting, it is believed that AAV toxicity may be due to systemic effects such as hepatotoxicity, platelet activation and loss, and complement activation and loss. All of these toxicities and others may be reduced, minimized, or completely avoided via the loco-regional perfusate application described in the methods and systems disclosed herein. As such, doses up to about 5×10¹⁵ vg/kg heart tissue may be well tolerated. In certain embodiments, AAV doses to the heart, expressed as vg/kg heart tissue, may exceed the highest systemically administered doses by a factor of about 2 to about 200, about 5 to about 150, about 10 to about 100, or any sub-range therein.

Apart from viral vectors, non-viral expression constructs may also be used for introducing a gene encoding a target protein or a functioning variant or fragment thereof into a cell of a patient. Non-viral expression vectors which permit the in vivo expression of protein in the target cell include, for example, a plasmid, a modified RNA, a cDNA, antisense oligomers, DNA-lipid complexes, nanoparticles, exosomes, any other non-viral shuttle suitable for gene therapy, variations thereof, and a combination thereof.

Apart from viral vectors and non-viral expression vectors, nuclease systems may also be used, in conjunction with a vector and/or an electroporation system, to enter into a cell of a patient and introduce therein a gene encoding a target protein or a functioning variant or fragment thereof. Exemplary nuclease systems may include, without limitations, a clustered regularly interspaced short palindromic repeats (CRISPR), a DNA cutting enzyme (e.g., Cas9), meganucleases, TALENs, zinc finger nucleases, any other nuclease system suitable for gene therapy, variations thereof, and a combination thereof. For instance, in one embodiment, one viral vector (e.g., AAV) may be used for a nuclease (e.g., CRISPR) and another viral vector (e.g., AAV) may be used for a DNA cutting enzyme (e.g., Cas9) to introduce both (the nuclease and the DNA cutting enzyme) into a target cell.

Other vector delivery systems which can be employed to deliver a therapeutic polynucleotide sequence encoding a therapeutic gene into cells are receptor-mediated delivery vehicles. These take advantage of the selective uptake of macromolecules by receptor-mediated endocytosis in almost all eukaryotic cells. Because of the cell type-specific distribution of various receptors, the delivery can be highly specific. Receptor-mediated gene targeting vehicles may include two components: a cell receptor-specific ligand and a DNA-binding agent.

Suitable methods for the transfer of non-viral vectors into target cells are, for example, the lipofection method, the calcium-phosphate co-precipitation method, the DEAE-dextran method and direct DNA introduction methods using micro-glass tubes, ultrasound, electroporation, and the like. Prior to the introduction of the vector, the cardiac muscle cells may be treated with a permeabilization agent, such as phosphatidylcholine, streptolysins, sodium caprate, decanoylcarnitine, tartaric acid, lysolecithin, Triton X-100, and the like. Exosomes may also be used to transfer naked DNA or AAV-encapsidated DNA.

A gene therapy vector of the invention may comprise a promoter that is functionally linked to the nucleic acid sequence encoding to the target protein. The promoter sequence must be compact and ensure a strong expression. Preferably, the promoter provides for an expression of the target protein in the myocardium of the patient that has been treated with the gene therapy vector. In some embodiment, the gene therapy vector comprises a cardiac-specific promoter which is operably linked to the nucleic acid sequence encoding the target protein. As used herein, a "cardiac-specific promoter" refers to a promoter whose activity in cardiac cells is at least 2-fold higher than in any other non-cardiac cell type. Preferably, a cardiac-specific promoter suitable for being used in the vector of the invention has an activity in cardiac cells which is at least 5-fold, at least 10-fold, at least 15-fold, at least 20-fold, at least 25-fold, or at least 50-fold higher compared to its activity in a non-cardiac cell type.

The cardiac-specific promoter may be a selected human promoter, or a promoter comprising a functionally equivalent sequence having at least about 80%, at least about 90%, at least about 95%, at least about 96%, at least about 97%, at least about 98%, or at least about 99% sequence identity to the selected human promoter. An exemplary non-limiting promoter that may be used is a cardiac troponin T promoter (TNNT2). Other non-limiting examples of promoters include alpha myosin heavy chain promoter, the myosin light chain 2v promoter, the alpha myosin heavy chain promoter, the alpha-cardiac actin promoter, the alpha-tropomyosin promoter, the cardiac troponin C promoter, the cardiac troponin I promoter, the cardiac myosin-binding protein C promoter, and the sarco/endoplasmic reticulum Ca²⁺-ATPase (SERCA) promoter (e.g., isoform 2 of this promoter (SERCA2)).

The vectors useful in the present invention may have varying transduction efficiencies. As a result, the viral or non-viral vector transduces more than, equal to, or at least about 10%, about 20%, about 30%, about 40%, about 50%, about 55%, about 60%, about 65%, about 70%, about 75%, about 80%, about 85%, about 90%, about 95%, about 99%, or 100% of the cells of the targeted vascular territory. More than one vector (viral or non-viral, or combinations thereof) can be used simultaneously or in sequence. This can be used to transfer more than one polynucleotide, and/or target more than one type of cell. Where multiple vectors or multiple agents are used, more than one transduction/transfection efficiency can result.

Pharmaceutical compositions that contain gene therapy vectors may be prepared either as liquid solutions or suspensions. The pharmaceutical composition of the invention can include commonly used pharmaceutically acceptable excipients, such as diluents and carriers. In particular, the composition comprises a pharmaceutically acceptable carrier, e.g., water, saline, Ringer's solution, or dextrose solution. In addition to the carrier, the pharmaceutical composition may also contain emulsifying agents, pH buffering agents, stabilizers, dyes, and the like.

In certain embodiments, a pharmaceutical composition will comprise a therapeutically effective gene dose, which is a dose that is capable of preventing or treating cardiomyopathy in a subject, without being toxic to the subject. Prevention or treatment of cardiomyopathy may be assessed as a change in a phenotypic characteristic associated with cardiomyopathy with such change being effective to prevent or treat cardiomyopathy. Thus, a therapeutically effective gene dose is typically one that, when administered in a physiologically tolerable composition, is sufficient to improve or prevent the pathogenic heart phenotype in the treated subject.

Heart conditions that may be treated by the methods disclosed herein may include, without limitations, one or more of a genetically determined heart disease (e.g., genetically determined cardiomyopathy), arrhythmic heart disease, heart failure, ischemia, arrhythmia, myocardial infarction, congestive heart failure, transplant rejection, abnormal heart contractility, non-ischemic cardiomyopathy, mitral valve regurgitation, aortic stenosis or regurgitation, abnormal Ca²⁺ metabolism, congenital heart disease, primary or secondary cardiac tumors, and combinations thereof.

### ILLUSTRATIVE EXAMPLES

The following example is set forth to assist in understanding the disclosure and should not, of course, be construed as specifically limiting the embodiments described and claimed herein. Such variations of the embodiments, including the substitution of all equivalents now known or later developed, which would be within the purview of those skilled in the art, and changes in formulation or minor changes in experimental design, are to be considered to fall within the scope of the embodiments incorporated herein.

### Example 1: Feasibility study of loco-regional perfusion in three pigs

Feasibility of the LRP system established by successfully performing the procedure for 60 minutes in three pigs (sus scrofa domestica). In two pigs, a thoracotomy was performed for surveillance purposes, but all catheters were introduced percutaneously. In the third pig, no thoracotomy was performed and the entire LRP procedure was performed percutaneously.

LRP was performed on the three animals utilizing the LRP system 100 illustrated in and described with respect to FIGS. 1A and 1B. In all three animals, the LRP procedure could be maintained while the heart was spontaneously beating for 60 min without any technical problems. During LRP, all animals (n = 3) were hemodynamically stable without any need for inotropes. Post-LRP cardiac function was unremarkable and comparable to baseline for all animals. Overall occlusion of the coronary arteries was acceptable: in Animal 1, the LCA could not be fully occluded (leakage was considered mild), in Animal 2, the RCA could not be fully occluded (leakage was considered to be trace); and in Animal 3, both coronary arteries could be occluded.

The tight occlusion of the coronary sinus (CS) was technically more challenging due to the variable anatomy of the pig where, in contrast to humans, the vena azygos inserts directly into the coronary sinus and needs to be occluded to simulate the human situation. Full occlusion was achieved in Animal 3 (using a Reliant balloon), partial occlusion achieved in Animal 1 and Animal 2 (ProPledge catheters). Flow rates during 60 minutes of the LRP procedure ranging from 166 mL/min up to 244 mL/min could be achieved. Accessory devices that were used in this example are listed in , including their intended uses and the use in the LRP system in accordance with the embodiments of the disclosure.

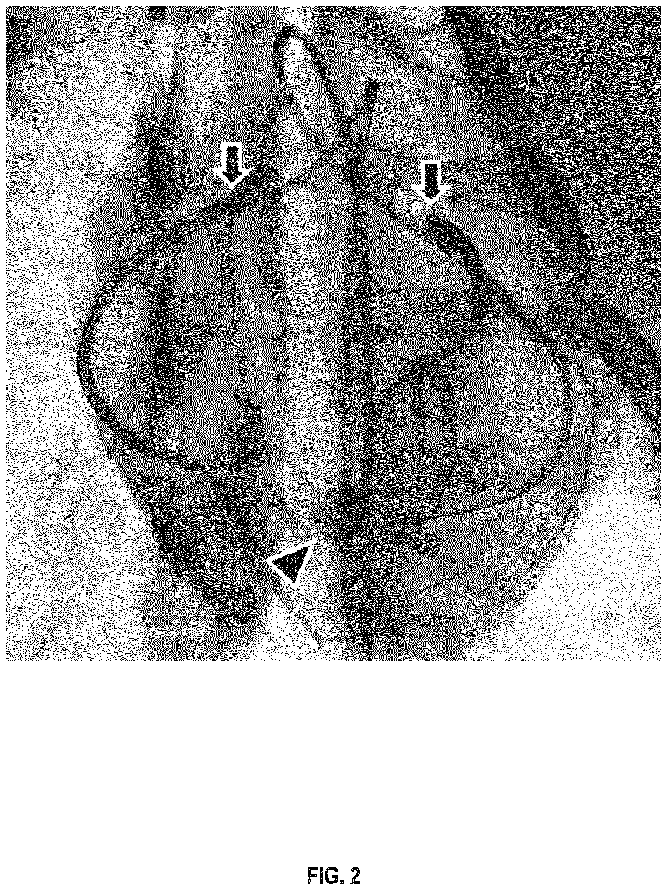

FIG. 2 is a radiograph of a representative LRP in situ setup, where coronary artery catheters are indicated by arrows and the coronary sinus balloon (collection catheter) is indicated by a triangle.

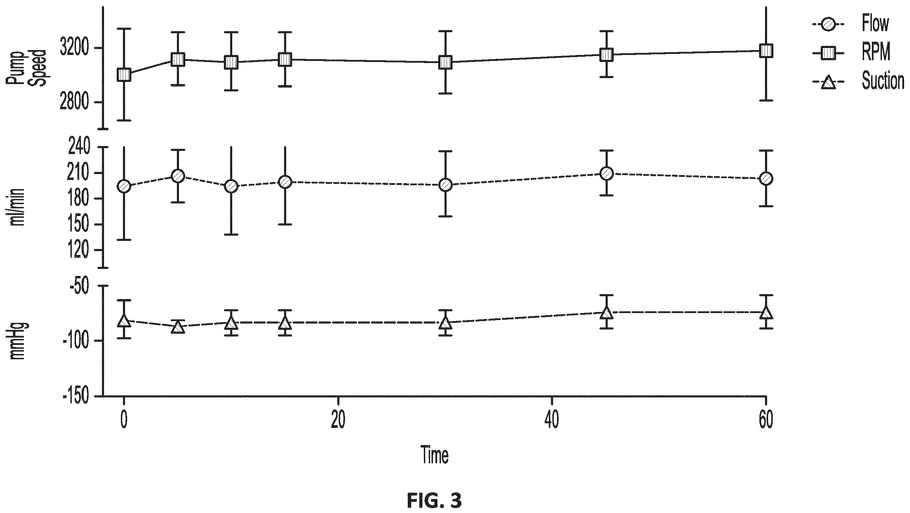

The mean values of LRP parameters (pump speed, flow, and pressure) for the three animals are summarized in Fig. 3 (with the bars representing standard deviation).

Table, including their intended uses and the use in the LRP system in accordance with the embodiments of the disclosure.

FIG. 2 is a radiograph of a representative LRP in situ setup, where coronary artery catheters are indicated by arrows and the coronary sinus balloon (collection catheter) is indicated by a triangle.

The mean values of LRP parameters (pump speed, flow, and pressure) for the three animals are summarized in Fig. 3 (with the bars representing standard deviation).

**Table 1: Devices used for LRP procedure**

| **Item** | **CE Intended Use** | **LRP Use** | **Brand** | **Study** |
|---|---|---|---|---|
| FlowGate² | Neurology thrombectomy | Coronary artery perfusion | Stryker | Animal 1 |
| | | | | Animal 2 |
| | | | | Animal 3 |
| ProPledge | Retrograde cardioplegia | Coronary sinus blood return | Edwards | Animal 1 |
| | | | | Animal 2 |
| | | | | Animal 3 |
| Endo Vent | Pulmonary artery blood venting (suction) | Coronary sinus blood return and azygos occlusion | Edwards | Animal 1 |
| | | | | Animal 2 |
| | | | | Animal 3 |
| D100 Oxygenator set | Child cardiopulmonary bypass (CPB) set | Blood oxygenation and tubing | Dideco-Livanova | Animal 1 |
| | | | | Animal 2 |
| | | | | Animal 3 |
| Revolution 5 | Centrifugal blood pump | Centrifugal blood pump | Sorin-Livanova | |
| Rotaflow RF-32 | Centrifugal blood pump | Centrifugal blood pump | Getinge | Animal 1 |
| | | | | Animal 2 |
| | | | | Animal 3 |
| BPX-80 | Centrifugal blood pump | Centrifugal blood pump | Medtronic | Safety study |
| Bio-Medicus 550 Bio-Console | ECMO pump console | ECMO pump console | Medtronic | Animal 1 |
| | | | | Animal 2 |
| | | | | Animal 3 |
| PressureWire X | Coronary pressure wire | Coronary pressure wire | Saint Jude Medical / Abbott | Animal 1 |
| | | | | Animal 2 |
| | | | | Animal 3 |
| Fractional flow reserve (FFR) console | FFR console | Coronary pressure console | Saint Jude Medical / Abbott | Animal 1 |
| | | | | Animal 2 |
| | | | | Animal 3 |
| Reliant | Aortic endo clamp | Coronary sinus occlusion | Medtronic | Animal 3 |
| Fogarty catheter | Thrombectomy | Coronay sinus occlusion | Fogarty | |

**Table 2: Flow and pressure characteristics over 60 minutes of the LRP procedure**

| **Animal 1** | | | | | | | |
|---|---|---|---|---|---|---|---|
| Time | 0 min | 5 min | 10 min | 15 min | 30 min | 45 min | 60 min |
| Flow (mL/min) | 140 | 200 | 190 | 200 | 180 | 220 | 190 |
| Pressure (mm/Hg) | -90 | -90 | -90 | -90 | -90 | -60 | -60 |
| RPM | 2650 | 3020 | 3020 | 3020 | 890 | 3060 | 2930 |
| Coronary pressure | 48/28 | 44/24 | 44/25 | 48/28 | 44/25 | 47/28 | 48/20 |
| Mean coronary pressure | 37 | 32 | 32 | 36 | 32 | 37 | 34 |
| Systemic pressure | 77/51 | 70/47 | 69/47 | 76/52 | 69/48 | 77/53 | 70/48 |
| Mean systemic pressure | 62 | 57 | 56 | 62 | 57 | 60 | 58 |
| | | | | | | | |

| **Animal 2** | | | | | | | |
|---|---|---|---|---|---|---|---|
| Time | 0 min | 5 min | 10 min | 15 min | 30 min | 45 min | 60 min |
| Flow (mL/min) | 180 | - | 140 | 150 | 170 | 180 | 180 |
| Pressure (mmHg) | -90 | - | -90 | -90 | -90 | -90 | -90 |
| RPM | 3020 | - | 2940 | 2980 | 3050 | 3050 | 3010 |
| Coronary pressure | 65/33 | - | 61/46 | 63/47 | 73/52 | 70/48 | 66/41 |
| Mean coronary pressure | 41 | - | 51 | 52 | 60 | 58 | 51 |
| Systemic pressure | 70/50 | - | 60/43 | 60/43 | 68/55 | 67/46 | 59/42 |
| Mean systemic pressure | 61 | - | 50 | 49 | 62 | 56 | 51 |
| Heart rate | 71 | - | 74 | 75 | 78 | 80 | 81 |
| | | | | | | | |

| **Animal 3** | | | | | | | |
|---|---|---|---|---|---|---|---|
| Time | 0 min | 5 min | 10 min | 15 min | 30 min | 45 min | 60 min |
| Flow (mL/min) | 260 | 240 | 250 | 250 | 240 | 230 | 240 |
| Pressure | -60 | -80 | -70 | -70 | -70 | -70 | -70 |
| RPM | 3330 | 3350 | 3350 | 3350 | 3350 | 3350 | 3610 |
| Coronary pressure | 54/44 | 58/44 | 53/4 | 52/41 | 50/40 | 50/40 | 86/56 |
| Mean coronary pressure | 49 | 49 | 47 | 46 | 45 | 42 | 70 |
| Systemic pressure | 71/47 | 66/43 | 67/45 | 67/45 | 67/39 | 54/40 | 57/7 |
| Mean systemic pressure | 57 | 54 | 54 | 54 | 47 | 47 | 30 |
| Heart rate | 73 | 74 | 73 | 72 | 77 | 97 | 88 |

### Example 2: Safety study of the loco-regional perfusion system in two pigs

Safety of the LRP system was established by performing the LRP procedure using a percutaneous approach for 60 minutes in two pigs (sus scrofa domestica) and following the animals for 24 hours after the procedure while the animals were kept under anaesthesia. Following the 24-hour period, the animals were sacrificed and a macroscopic and microscopic examination of their hearts was carried out. In addition, blood biomarkers were obtained to evaluate tissue damage of the heart.

In both experiments the LRP could be successfully performed and without any serious adverse effect. A technical issue occurred in the with Animal 5, where the pump head tubing connection failed after 10 minutes. LRP was immediately stopped, and all catheters were disengaged and deflated. The pump head was immediately replaced, and the LRP system was reconnected, deaired, and restarted. During this maneuver, the animal was hemodynamically stable, and no serious adverse effect was observed. The LRP procedure was then maintained for 60 minutes, thus demonstrating the safety efficacy even with minor equipment failures.

Throughout the procedures, including initiation, re-initiation of LRP, and up to 24 hours after, the animals were hemodynamically stable without any need for inotropes.

A mean LRP flow of 173 mL/min could be achieved while the left main coronary artery and the right coronary artery were fully occluded and the coronary sinus was partially occluded (leakage was moderate) for both animals. The post-operative and 24-hour cardiac functions were unremarkable and comparable to baseline. Cardiac biomarkers (myoglobin and troponin) only slightly increased during and shortly after the LRP procedure, but then immediately dropped towards baseline values during 24-hour follow up. Given the continuous hemodynamic stability of the animals throughout the entire procedure, the absence of serious adverse effects, and only minor and temporary increases of cardiac biomarkers, as well as only temporary electrocardiogram changes with immediate normalization during the 24 hours, the LRP procedure was demonstrated to be safe. Table 3 compiles the flow and pressure characteristics over 60 minutes of the LRP procedure in the Animal 4 and Animal 5 used in the safety study.

**Table 3: Parameters for safety study**

| **Animal 4** | | | | | |
|---|---|---|---|---|---|
| Time | 0 | 15 min | 30min | 45min | 60min |
| Flow (mL/min) | 180-190 | 200 | 180 | 180 | 190 |
| Suction | | -70 | -70 | -70 | -70 |
| RPM | | 3400 | 3400 | 3320 | 3350 |
| Coronary pressure | | 93/52 | 90/63 | 88/37 | 82/43 |
| Mean | | 74 | 77 | 62 | 65 |
| Systemic pressure | | 104/65 | 100/64 | 94/61 | 94/56 |
| Mean | | 80 | 78 | 77 | 65 |
| | | | | | |

| **Animal 5** | | | | | |
|---|---|---|---|---|---|
| Time | 5min | 15 min | 30min | 45min | 60min |
| Flow (mL/min) | 150 | 150 | 160 | 150 | 170 |
| Suction (mmHg) | -80 | -80 | -80 | -80 | -80 |
| RPM | 2390 | 2400 | 2440 | 2400 | 2410 |
| Coronary pressure | 76/44 | 80/40 | 81/40 | 90/45 | 72/24 |
| Mean | 61 | 59 | 58 | 62 | 43 |
| Systemic | 86/52 | 86/53 | 80/46 | 100/62 | 84/47 |
| Mean | 66 | 66 | 60 | 77 | 63 |

The hearts of Animal 4 and Animal 5 were macroscopically examined following sacrifice.

The heart weight for Animal 4 was 312 grams. No gross pathology was observed, and in particular there were no signs of myocardial ischemia or myocardial infarction. On the posterior side of the heart of Animal 4, a localized hematoma was observed in the area of the right coronary artery, most likely due to wire injury during the procedure.

The heart weight for Animal 5 was 293 grams. No gross pathology was observed, and in particular there were no signs of myocardial ischemia or myocardial infarction. On the posterior side of the heart of Animal 5, localized hematomas were observed in the areas of the distal right coronary artery and distal left circumflex artery, most likely due to wire injury during procedure.

In order to ascertain the biochemical integrity of the heart tissue, seric cardiac biomarkers were obtained, and summarized in Table 4. Creatine kinase (CK) levels remained stable during the LRP procedure but showed a continuous rise post-LRP most likely due to the animal lying in the supine position. Myoglobin levels remained stable during the LRP procedure and showed only minimal increase thereafter, still within reference levels for Animal 4 and only very slightly above reference levels for Animal 5. Troponin T increased minimally during the LRP procedure with a peak at 60 minutes followed by a drop to baseline values during follow up.

**Table 4: Seric biomarkers (creatine kinase (CK), myoglobin (Myo), and troponin T (Trop)) values were obtained at baseline, at 30 min and 60 min during LRP, and at various intervals post-LRP**

| **Animal 4** | Ref. value | Baseline | LRP 30min | LRP 60min | 4h | 8h | 12h | 16h | 20h | 24h |
|---|---|---|---|---|---|---|---|---|---|---|
| CK | <190U/ L | 871 | 865 | 883 | 937 | 1293 | 1285 | 1270 | 2068 | 2640 |
| Myo. | 25-72µg/L | <21 | <21 | <21 | <21 | <21 | <21 | 24 | 35 | 58 |
| Trop. | < 14ng/L | 53 | 85 | 106 | 56 | 45 | 34 | 28 | 32 | 53 |

| **Animal 5** | Ref. value | Baseline | LRP 30min | LRP 60min | 4h | 8h | 12h | 16h | 20h | 24h |
|---|---|---|---|---|---|---|---|---|---|---|
| CK | <190U/L | 988 | 899 | 931 | 1946 | 2943 | 4064 | 4929 | 5496 | 7655 |
| Myo. | 25-72µg/L | <21 | <21 | <21 | <21 | 26 | 35 | 37 | 43 | 83 |
| Trop. | <14ng/L | 37 | 44 | 66 | 138 | 120 | 93 | 72 | 61 | 58 |

In the foregoing description, numerous specific details are set forth, such as specific materials, dimensions, processes parameters, etc., to provide a thorough understanding of the present invention. The particular features, structures, materials, or characteristics may be combined in any suitable manner in one or more embodiments. The words "example" or "exemplary" are used herein to mean serving as an example, instance, or illustration. Any aspect or design described herein as "example" or "exemplary" is not necessarily to be construed as preferred or advantageous over other aspects or designs. Rather, use of the words "example" or "exemplary" is simply intended to present concepts in a concrete fashion. As used in this application, the term "or" is intended to mean an inclusive "or" rather than an exclusive "or". That is, unless specified otherwise, or clear from context, "X includes A or B" is intended to mean any of the natural inclusive permutations. That is, if X includes A; X includes B; or X includes both A and B, then "X includes A or B" is satisfied under any of the foregoing instances. Reference throughout this specification to "an embodiment", "certain embodiments", or "one embodiment" means that a particular feature, structure, or characteristic described in connection with the embodiment is included in at least one embodiment. Thus, the appearances of the phrase "an embodiment", "certain embodiments", or "one embodiment" in various places throughout this specification are not necessarily all referring to the same embodiment.

The present invention has been described with reference to specific exemplary embodiments thereof. The specification and drawings are, accordingly, to be regarded in an illustrative rather than a restrictive sense. Various modifications of the invention in addition to those shown and described herein will become apparent to those skilled in the art and are intended to fall within the scope of the appended claims.

Some embodiments of the invention are disclosed in the following items:
1. A method of perfusing a drug in an unarrested beating heart of a patient comprising:
   positioning a first drug delivery catheter in the right coronary artery of the heart;
   positioning a second drug delivery catheter in the left main coronary artery of the heart;
   positioning a drug collection catheter in the coronary sinus of the heart, wherein the first drug delivery catheter, the second drug delivery catheter, and the drug collection catheter together with the coronary arteries of the heart, the coronary venous system of the heart, and a membrane oxygenation device form a closed circuit; and
   perfusing the drug through the closed circuit, wherein the closed circuit isolates the coronary circulation of the patient from the systemic circulation of the patient.
2. The method of item 1, further comprising applying negative pressure at the drug collection catheter.
3. The method of item 2, wherein the negative pressure ranges from about -100 mmHg to 0 mmHg.
4. The method of any one of the preceding items, wherein one or more of the first drug delivery catheter, the second drug delivery catheter, or the drug collection catheter are introduced percutaneously.
5. The method of any one of the preceding items, wherein the first drug delivery catheter and/or the second drug delivery catheter are positioned via antegrade intubation.
6. The method of any one of the preceding items, wherein the first drug delivery catheter and/or the second drug delivery catheter are positioned via the aorta of the patient by accessing the aorta femoralis and/or the aorta radialis.
7. The method of any one of the preceding items, wherein the drug collection catheter is positioned in the coronary sinus via the vena cava of the patient.
8. The method of any one of the items 1-4, wherein the drug collection catheter is positioned via the vena jugularis of the patient or the vena femoralis.
9. The method of any one of the preceding items, wherein the membrane oxygenation device is positioned between the collection catheter and one or more of the first drug delivery catheter and the second drug delivery catheter.
10. The method of any one of the preceding items, further comprising circulating blood through the closed circuit.
11. The method of item 10, wherein the blood comprises autologous blood, matched blood from donors, or a combination thereof.
12. The method of any one of items 10-11, wherein blood components such as serum or plasma are chosen according to one or more parameter, wherein the one or more parameters comprise presence or absence of selected antibodies.
13. The method of any one of items 10-12, wherein about 1000 mL, about 800 mL, about 600 mL, about 400 mL, about 200 mL, about 100 mL, or about 50 mL of blood is circulated through the closed circuit.
14. The method of any one of the preceding items, wherein the perfusing occurs over a duration of about 5 minutes to about 5 hours, about 15 minutes to about 4 hours, about 30 minutes to about 3 hours, or about 1 hour to about 2 hours.
15. The method of any one of the preceding items, wherein the perfusing occurs for at least 60 minutes.
16. The method of any one of the preceding items, wherein the perfusing occurs at a flow rate of about 75 mL/min to about 750 mL/min, about 150 mL/min to about 500 mL/min, or about 200 mL/min to about 300 mL/min.
17. The method of any one of the preceding items, wherein the drug is suitable for treatment of a heart condition.
18. The method of item 17, wherein the heart condition is heart failure.
19. The method of item 17, wherein the heart condition is a genetically determined heart disease.
20. The method of item 19, wherein the genetically determined heart disease is a genetically determined cardiomyopathy.
21. The method of any one of the preceding items, wherein the drug comprises a therapeutic polynucleotide sequence.
22. The method of item 21, wherein the therapeutic polynucleotide sequence is present in one or more viral vectors.
23. The method of item 22, wherein the one or more viral vectors is selected from the group consisting of an adeno-associated virus, an adenovirus, a retrovirus, a herpes simplex virus, a bovine papilloma virus, a lentiviral vector, a vaccinia virus, a polyoma virus, a sendai virus, orthomyxovirus, paramyxovirus, papovavirus, picornavirus, pox virus, alphavirus, variations thereof, and combinations thereof.
24. The method of item 23, wherein the viral vector is an adeno-associated virus (AAV).
25. The method of item 24, wherein the AAV is one or more of AAV 1, AAV2, AAV3, AAV4, AAV5, AAV6, AAV7, AAV8, AAV9, AAV10, AAV11, AAV12, variations thereof, and combinations thereof.
26. The method of any one of items 21-25, wherein the therapeutic polynucleotide sequence comprises a nucleic acid sequence encoding to a protein for treatment of a heart condition.
27. The method of item 26, wherein the protein corresponds to a gene expressed in a human heart.
28. The method of item 27, wherein the protein is one or more of SERCA2, MyBPC3, MYH7, PKP2, dystrophin, FKRP, or a combination or variation thereof.
29. The method of any one of items 21-28, wherein the therapeutic polynucleotide sequence comprises a promoter.
30. The method of any one of the preceding items, wherein less than about 20% v/v, less than about 15% v/v, less than about 10% v/v, less than about 5% v/v, less than about 4% v/v, less than about 3% v/v, less than about 2% v/v, less than about 1% v/v, less than about 0.5% v/v, or substantially no (0% v/v) blood circulated through the closed circuit leaks outside of the closed circuit.
31. The method of any one of the preceding items, wherein less than about 20% v/v, less than about 15%v/v, less than about 10% v/v, less than about 5% v/v, less than about 4% v/v, less than about 3% v/v, less than about 2% v/v, less than about 1% v/v, less than about 0.5% v/v, or substantially no (0% v/v) drug perfused through the closed circuit leaks outside of the closed circuit.
32. The method of any one of the preceding items, wherein one or more of the first drug delivery catheter, the second drug delivery catheter, or the drug collection catheter is a balloon catheter.
33. A method of maintaining perfusion of a perfusate through a closed circuit in a heart of a patient, wherein the heart is unarrested and beating during the perfusion, the method comprising:
   positioning a first catheter in the right coronary artery of the heart;
   positioning a second catheter in the left main coronary artery of the heart;
   positioning a collection catheter in the coronary sinus of the heart, wherein the first catheter, the second catheter, and the collection catheter together with the coronary arteries, the coronary venous system, and a membrane oxygenation device form the closed circuit through the heart; and
   flowing the perfusate through the closed circuit by introducing the perfusate into the heart via the first catheter and the second catheter and collecting the perfusate via the collection catheter, wherein the closed circuit isolates the coronary circulation of the patient from the systemic circulation of the patient.
34. The method of item 33, wherein the perfusion is maintained for at least 60 minutes.
35. The method of item 34, wherein the perfusion is maintained for at least 120 minutes.
36. The method of any of items 33-35, further comprising applying negative pressure at the collection catheter, wherein the negative pressure ranges from about -100 mmHg to 0 mmHg.
37. The method of any of items 33-36, wherein one or more of the first catheter, the second catheter, or the collection catheter are introduced percutaneously.
38. The method of any of items 33-37, wherein the membrane oxygenation device is positioned between the collection catheter and one or more of the first drug delivery catheter and the second drug delivery catheter.
39. The method of any of items 33-38, further comprising circulating blood through the closed circuit, wherein the blood comprises autologous blood, matched blood from donors, or a combination thereof.
40. The method of item 39, wherein about 1000 mL, about 800 mL, about 600 mL, about 400 mL, about 200 mL, about 100 mL, or about 50 mL of blood is circulated through the closed circuit.
41. The method of any of items 33-40, wherein the perfusing occurs at a flow rate of about 75 mL/min to about 750 mL/min, about 150 mL/min to about 500 mL/min, or about 200 mL/min to about 300 mL/min.
42. The method of any of items 33-41, wherein less than about 20% v/v, less than about 15% v/v, less than about 10% v/v, less than about 5% v/v, less than about 4% v/v, less than about 3% v/v, less than about 2% v/v, less than about 1% v/v, less than about 0.5% v/v, or substantially no (0% v/v) blood circulated through the closed circuit leaks outside of the closed circuit.
43. The method of any of items 33-42, wherein one or more of the first catheter, the second catheter, or the collection catheter is a balloon catheter.
44. A system for performing loco-regional perfusion within the heart of a patient when fluidly coupled thereto, the system comprising:
   a first catheter adapted for insertion into the right coronary artery of the heart;
   a second catheter adapted for insertion into the left main coronary artery of the heart;
   a collection catheter adapted for insertion into the coronary sinus of the heart;
   a membrane oxygenation device fluidly coupled to the first catheter, the second catheter, the collection catheter, and an oxygen source, wherein the first catheter, the second catheter, the collection catheter, and the membrane oxygenation device together form a closed circuit through the heart that is isolated from the patient's systemic circulation when the first catheter is inserted into the right coronary artery, the second catheter is inserted into the left main coronary artery, and the collection catheter is inserted into the coronary sinus; and
   a pump configured to drive fluid flow through the first catheter and the second catheter.
45. A loco-regional perfusion system comprising:
   a first catheter inserted into the right coronary artery of a heart of a patient;
   a second catheter inserted into the left main coronary artery of the heart;
   a collection catheter inserted into the coronary sinus of the heart; and
   a membrane oxygenation device fluidly coupled to the first catheter, the second catheter, the collection catheter, and an oxygen source, wherein the first catheter, the second catheter, the collection catheter, and the membrane oxygenation device together with the coronary arteries and the coronary venous system of the heart form a closed circuit through the heart that is isolated from the patient's systemic circulation; and
   a pump configured to drive fluid flow into the heart via the first catheter and the second catheter and out of the heart via the collection catheter.
46. The loco-regional perfusion system of either item 44 or item 45, wherein the membrane oxygenation device comprises a reservoir configured for injecting a drug into the closed circuit during perfusion.
47. The loco-regional perfusion system of any of items 44-46, wherein the pump is configured to generate negative pressure ranges from about -100 mmHg to 0 mmHg.
48. The loco-regional perfusion system of any of items 44-47, wherein one or more of the first drug delivery catheter, the second drug delivery catheter, or the drug collection catheter are introduced percutaneously.
49. The loco-regional perfusion system of items 44-48, wherein the first drug delivery catheter and/or the second drug delivery catheter are positioned via antegrade intubation.
50. The loco-regional perfusion system of items 44-49, wherein the drug collection catheter is positioned in the coronary sinus via the vena cava of the patient.
51. The loco-regional perfusion system of either item 44 or item 45 configured to perform any of the methods of items 1-43.

## Claims

1. Components configured for use in performing loco-regional perfusion within the heart of a patient when fluidly coupled thereto, the components comprising:
a first drug-delivery catheter adapted for insertion into the right coronary artery of the heart;
a second drug-delivery catheter adapted for insertion into the left main coronary artery of the heart; and
a collection catheter adapted for insertion into the coronary sinus of the heart,
wherein each of the first drug-delivery catheter, the second drug-delivery catheter, and the collection catheter are configured to fluidly couple to a membrane oxygenation device to create a closed circuit through the heart that is substantially isolated from the patient's systemic circulation when the first drug-delivery catheter is inserted into the right coronary artery, the second drug-delivery catheter is inserted into the left main coronary artery, and the collection catheter is inserted into the coronary sinus.

2. The components of claim 1, further comprising a reservoir configured for injecting a drug into a perfusate of the closed circuit, wherein the drug is suitable for treatment of a heart condition, wherein the heart condition is preferably a genetically determined heart disease, and wherein the genetically determined heart disease is preferably a genetically determined cardiomyopathy.

3. The components of either claim 1 or claim 2, wherein the drug comprises a therapeutic polynucleotide sequence.

4. The components of claim 3, wherein the therapeutic polynucleotide sequence is present in one or more viral vectors.

5. The components of claim 4, wherein at least one of the one or more viral vectors is an adeno-associated virus (AAV).

6. The components of claim 5, wherein the AAV is a serotype selected from AAV1, AAV2, AAV3, AAV4, AAV5, AAV6, AAV7, AAV8, AAV9, AAV10, AAV11, AAV12, variants thereof, and combinations thereof.

7. The components of any one of claims 3-6, wherein the therapeutic polynucleotide sequence encodes a protein selected from SERCA2, MyBPC3, MYH7, PKP2, dystrophin, FKRP, or functional variants thereof.

8. The components of any one of claims 3-7, wherein the therapeutic polynucleotide sequence comprises a promoter.

9. The components of any one of the preceding claims, wherein one or more of the first drug-delivery catheter, the second drug-delivery catheter, or the collection catheter is a balloon catheter.

10. The components of any one of the preceding claims, wherein the first drug-delivery catheter and/or the second drug-delivery catheter is configured for positioning via antegrade intubation.

11. The components of any one of the preceding claims, wherein the collection catheter is configured for positioning in the coronary sinus via the vena cava of the patient.

12. The components of any one of the preceding claims, wherein the components are configured for carrying out a method comprising:
positioning the first drug delivery catheter in the right coronary artery of the heart;
positioning the second drug delivery catheter in the left main coronary artery of the heart;
positioning the collection catheter in the coronary sinus of the heart; and
fluidly coupling the first drug delivery catheter, the second drug delivery catheter and the collection catheter to a membrane oxygenation device.

13. The components of claim 12, wherein the method further comprises fluidly coupling a pump to the membrane oxygenation device.

14. The components of claim 13, wherein the method further comprises driving, via the pump, a drug through the first drug delivery catheter and the second drug delivery catheter.

15. The components of claim 14, wherein the method further comprises maintaining perfusion through the closed circuit for at least 60 minutes.
